# EUROPÄISCHE PATENTSCHRIFT

(11) **EP 0 765 670 B1**
(45) Veröffentlichungstag und Bekanntmachung des Hinweises auf die Patenterteilung: **15.10.2003**
(21) Anmeldenummer: 96115020.8
(22) Anmeldetag: 19.09.1996
(51) Int. Cl.: A61M 1/16

(54) **Vorrichtung zur Überwachung der Funktionssicherheit der Membran eines Dialysators**
Means for controlling the integrity of the membrane of a dialyzer
Dispositif de contrôle de la sécurité d'emploi de la membrane d'un dialysateur

(30) Priorität: 27.09.1995 US 534495
(43) Veröffentlichungstag der Anmeldung: 02.04.1997
(73) Patentinhaber: Fresenius Medical Care Deutschland GmbH, 61352 Bad Homburg v.d.H. (DE)
(72) Erfinder: Folden, Thomas I., Alamo, CA. 94597. (US); Polaschegg, Hans D., 61440 Oberursel (DE); Peter, Harald, 61440 Oberursel (DE)
(74) Vertreter: Luderschmidt, Schüler & Partner GbR

(56) Entgegenhaltungen:
- WO-A-94/11093

## Beschreibung

Die Erfindung betrifft eine Vorrichtung in Verbindung mit einem Hämodialysegerät mit einer Ultrafiltrationseinrichtung, die es erlaubt, die Funktionssicherheit des Dialysators des Hämodialysegeräts während einer Hämodialysebehandlung zu überwachen.

Die Hämodialyse stellt ein Behandlungsverfahren beim chronischen Nierenversagen dar. Bei der Hämodialyse wird das Blut des Patienten außerhalb des Körpers in einer künstlichen Niere gereinigt, die auch als Dialysator bezeichnet wird. Der Dialysator weist eine Blutkammer und eine Dialysierflüssigkeitskammer auf, die von einer semipermeablen Membran getrennt werden. Während der Behandlung fließt Blut des Patienten durch die Blutkammer auf der einen Seite der Membran, während Dialysierflüssigkeit durch die Dialysierflüssigkeitskammer auf der anderen Seite der Membran strömt. Die Membran besteht im allgemeinen aus Platten oder Hohlfasern und hat eine Gesamtoberfläche von 1 bis 2 m². Die Integrität der Membran gewährleistet eine Trennung des Bluts von der Dialysierflüssigkeit. Aufgrund des Konzentrationsgefälles zwischen der Blutseite und der Dialysierflüssigkeitsseite treten harnpflichtige Substanzen aus dem Blut in die Dialysierflüssigkeit (Diffusion). Um das Blut effektiv von den harnpflichtigen Substanzen zu reinigen, wird die Dialysierflüssigkeitskammer kontinuierlich von frischer Dialysierflüssigkeit durchströmt.

Im Verlauf der Hämodialysebehandlung wird neben der Entfernung von harnpflichtigen Stoffen mit Hilfe der Diffusion dem Blut im allgemeinen auch Flüssigkeit entzogen. Zum Entzug von Flüssigkeit ist es erforderlich, zwischen der Blutseite und der Dialysierflüssigkeitsseite der Membran eine Druckdifferenz mittels einer Saugeinrichtung zu erzeugen. Dieser Vorgang wird als Ultratfiltration bezeichnet. Ein Hämodialysegerät mit einer derartigen Ultrafiltrationseinrichtung ist beispielsweise aus der DE-A-22 59 787 bekannt.

Zum Schutz des Patienten weisen die bekannten Dialysegeräte verschiedene Sicherheitssysteme auf. Ein bekanntes Sicherheitssystem umfaßt einen Blutleckdetektor, der die Membran des Dialysators auf Leckstellen überwacht, die dazu führen, daß Blut aus dem Blutkreislauf in den Dialysierflüssigkeitskreislauf gelangt. Die Detektion von Blut in der Dialysierflüssigkeit erfolgt bei den bekannten Blutleckdetektoren im allgemeinen mittels eines foto-optischen Sensors, mit dem sich die Konzentration von Blut in der Dialysierflüssigkeit bestimmen läßt. Die Blutleckdetektoren werden vielfach im Durchfluß betrieben und erzeugen ein Signal, wenn ein vorgegebener Grenzwert überschritten wird. Im Falle eines Blutlecks können verschiedene Maßnahmen eingeleitet werden. Es kann ein Alarm ausgelöst werden und die Stromversorgung der Blutpumpe kann unterbrochen werden. In einigen Systemen wird auch der Dialysator mittels einer Bypassleitung überbrückt.

Blutleckdetektoren sind in verschiedenen Ausführungsformen bekannt. US-A-4,181,610 beschreibt beispielsweise einen Blutleckdetektor mit einer optischen Meßzelle, die von der Dialysierflüssigkeit während der Testphase durchströmt wird. Auf der einen Seite der Meßzelle sind zwei Leuchtdioden angeordnet, und auf der anderen Seite der Meßzelle befindet sich ein fotooptischer Sensor. Eine der Leuchtdioden emittiert Licht mit einer längeren Wellenlänge, während die andere Leuchtdiode Licht mit einer kürzeren Wellenlänge emittiert. Beide Dioden werden derart betrieben, daß der fotooptische Sensor Signale der gleichen Stärke erzeugt, wenn sich in der Meßzelle kein Blut befindet. Die Signale des fotooptischen Sensors werden empfangen und ausgewertet, um Signale zu erzeugen, die in Abhängigkeit von der Größe des Blutlecks, falls ein solches auftritt, in eine Richtung abgelenkt werden. Wenn Luftblasen detektiert werden, führen diese zu Signalen in der anderen Richtung, was darauf zurückzuführen ist, daß Blut Licht mit kürzerer Wellenlänge stärker absorbiert, während Luftblasen Licht mit längerer Wellenlänge stärker absorbieren.

US-A-4,017,190 beschreibt ein System zur Detektion kleiner Mengen von Hämoglobin in einer Lösung, indem das Verhältnis zwischen der Lichttransmission bei unterschiedlichen Wellenlängen in einer Probenlösung erfaßt wird, wobei eine dieser Wellenlängen sich in einer Größenordnung bewegt, bei der Hämoglobin Licht stark absorbiert. Da es auf das Verhältnis der Signale ankommt, ist eine Messung mit hoher Sensitivität und Linearität möglich, wenn eine Verunreinigung und Trübung in der Probe oder in dem System vorliegt.

Aus US-A-3,832,067 ist ein Kolorimeter zur Detektion eines Blutlecks in einem Dialysegerät bekannt. Das Kolorimeter erfaßt das Vorliegen einer Verunreinigung, die für Licht einer bestimmten Farbe undurchlässig ist, das in die Flüssigkeitströmung eintritt. Wenn die Flüssigkeit durch das Kolorimeter strömt, wird eine Fotozelle auf einer Seite des Flüssigkeitsstroms nur mit Licht der bestimmten Farbe beleuchtet, das von der gegenüberliegenden Seite des Flüssigkeitsstroms herrührt, wobei die Fotozelle eine Veränderung in der Intensität des durchtretenden Lichts detektiert, die auf das Vorliegen einer Verunreinigung zurückzuführen ist.

US-A-3,900,396 schlägt einen Blutleckdetektor vor, der aus einer transparenten Leitung besteht, durch die Dialysierflüssigkeit durch einen Block zirkuliert. Der Block enthält eine Lampe, deren Licht durch einen ersten Kanal in dem Block, der die Leitung schräg schneidet und einen zweiten Kanal in dem Block tritt. Der zweite Kanal hat die gleiche Länge wie der erste Kanal und verläuft schräg zum ersten Kanal, ohne diesen zu schneiden. An den beiden Enden der Kanäle sind identische 550 bis 560 nm Filter und Fotozellen angeordnet. Die Kanäle schaffen relativ weite Dispersionswinkel. Ein Schaltkreis ist vorgesehen, um das Differenzsignal der Fotozellen zu messen und dieses in Form einer Spannung auszudrücken. Wenn die Differenz zwischen dieser Spannung und einer Referenzspannung über einem bestimmten Grenzwert liegt, wird ein Alarm ausgelöst.

US-A-4,060,485 beschreibt ein Dialysegerät mit einem herkömmlichen Blutleckdetektor, der eine fotoelektrische Einrichtung zur Detektion von Blut in einer Salzlösung aufweist, was auf eine Leckage oder Störung in der Dialyseeinheit hinweist. Wenn eine Blutleckage detektiert wird, gibt der Blutleckdetektor ein elektrisches Signal an einen logischen Schaltkreis ab, der eine Signallampe oder eine akustische Alarmeinrichtung aktiviert, die anzeigt, daß eine Blutleckage aufgetreten ist.

Während der Dialysebehandlung ist die Druckdifferenz zwischen Blut und Dialysierflüssigkeit im allgemeinen über die gesamte Länge der Membran positiv. Kommt es dann zu einer Verletzung der Membran, so kann Blut in die Dialysierflüssigkeit und somit in den Abfluß gelangen. In diesem Fall sprechen die bekannten Blutleckdetektoren an, die im Dialysierflüssigkeitsweg angeordnet sind. Die bekannten Blutleckdetektoren haben jedoch den Nachteil, daß sich Leckstellen in der Membran während einer sogenannten High-Flux-Dialysebehandlung nicht feststellen lassen. Bei der Verwendung von Dialysatoren mit hoher Permeabilität, die auch als High-Flux-Dialysatoren bezeichnet werden, kann während der Dialysebehandlung ein Zustand auftreten, bei dem zwar ein Nettodruckgefälle von der Blutseite zur Dialysierflüssigkeitsseite besteht, sich aber im Dialysator Bereiche ausbilden, bei denen der Druck in der Dialysierflüssigkeit höher ist als der Druck im Blut. In diesen Bereichen findet eine sogenannte "Rückfiltration" statt, d.h. über den Dialysator gelangt "gefilterte" Dialysierflüssigkeit zum Patienten. Dies kann sich bei geeigneter Rückhalterate des Filtersystems durchaus positiv auf die Behandlungsqualität auswirken. Bei einer Verletzung der Membran jedoch kann während einer "Rückfiltration" ungefilterte Dialysierflüssigkeit in den Blutkreislauf gelangen. Wenn Dialysierflüssigkeit, die nicht durch geeignete Maßnahmen, z.B. Online-Filtration, steril ist, in den Blutweg gelangt, können schwerwiegende Komplikationen, z.B. pyrogene Reaktionen oder eine Bakteriämie auftreten. Dies kann mit den bekannten Blutleckdetektoren nicht verhindert werden.

Die WO 94/11093 beschreibt eine Dialysevorrichtung mit einem extrakorporalen Blutkreislauf, der von der Membran eines Dialysators vom Dialysierflüssigkeitssystem getrennt ist. Die Dialysevorrichtung verfügt über ein System zur automatischen Erkennung einer Situation, bei der Blut aus dem Blutkreislauf über die Dialysemembran in das Dialysierflüssigkeitssystem übertritt, wenn der Dialysator von Blut bzw. Dialysierflüssigkeit durchflossen wird.

Der Erfindung liegt die Aufgabe zugrunde, eine Vorrichtung anzugeben, mit der sich die Sicherheit eines Hämodialysegerätes weiter erhöhen läßt.

Die Lösung der Aufgabe erfolgt erfindungsgemäß mit den im Patentanspruch 1 angegebenen Merkmalen.

Die erfindungsgemäße Vorrichtung beruht darauf, daß die Integrität der Membran des Dialysators durch Steuerung des Dialysierflüssigkeitsflusses und der Ultrafiltrationsrate geprüft wird.

Zur Prüfung der Membran auf Leckstellen wird der Fluß der Dialysierflüssigkeit durch die Dialysierflüssigkeitskammer des Dialysators unterbrochen und die Ultrafiltrationsrate wird erhöht, um zu gewährleisten, daß sich an jeder Stelle der Membran ein Druckgradient vom Blut zur Dialysierflüssigkeit einstellt. Die dabei entzogene Flüssigkeitsmenge kann dem Gesamtentzugsvolumen zugerechnet werden. Wenn es nun aufgrund von Leckstellen in der Membran zu einem Übertritt von Blut in die Dialysierflüssigkeit kommt, kann die Blutleckage im Dialysierflüssigkeitsweg detektiert werden, sobald der Dialysierflüssigkeitsfluß wieder in Gang gesetzt wird. Die Flußunterbrechung führt zu einer Aufkonzentration, so daß sich das Blut mit dem bekannten Blutleckdetektoren im Dialysierflüssigkeitsweg nachweisen läßt.

Die Ultrafiltrationsrate wird vorzugsweise für einen Zeitraum von mindestens 20 Sekunden erhöht. Um zuverlässige Meßergebnisse zu erzielen, ist es jedoch ausreichend, wenn die Ultrafiltrationsrate für eine Zeitdauer von nicht mehr als 60 Sekunden erhöht wird. Mit einer Ultrafiltrationsrate von ≥ 500 ml/min ist gewährleistet, daß sich an jeder Stelle der Membran ein Druckgradient vom Blut zur Dialysierflüssigkeit einstellt.

Die erfindungsgemäße Vorrichtung zur Durchführung des Verfahrens weist eine Steuereinheit mit einem Zeitglied auf, das derart ausgebildet ist, daß der Fluß der Dialysierflüssigkeit durch die Dialysierflüssigkeitskammer des Dialysators für ein vorgegebenes Zeitintervall unterbrochen und die Ultrafiltrationsrate in dem vorgegebenen Zeitintervall auf einen vorbestimmten Wert erhöht werden kann. Die Detektion der Blutleckage im Dialysierflüssigkeitsweg erfolgt mittels eines Blutleckdetektors, dessen Ausgangssignal von einer Auswerteinheit überwacht wird.

Bei einer bevorzugten Ausführungsform wird der Fluß der Dialysierflüssigkeit durch die Dialysierflüssigkeitskammer des Dialysators dann unterbrochen, wenn auf der Blutseite ein gegenüber der Dialysatseite der Membran negativer Transmembrandruck auftritt. Da die Testphase automatisch eingeleitet wird, kann verhindert werden, daß im Falle einer Rückfiltration Blut in den Dialysierflüssigkeitsweg gelangt. Es ist aber auch möglich, daß der Fluß der Dialysierflüssigkeit in vorgegebenen Zeitintervallen periodisch unterbrochen wird.

Im folgenden wird unter Bezugnahme auf die Zeichnungen ein Ausführungsbeispiel der Erfindung näher erläutert.

Es zeigen:
- Fig. 1: die wesentlichen Elemente eines Hämodialysegerätes mit der erfindungsgemäßen Vorrichtung zur Überwachung der Funktionssicherheit der Membran des Dialysators in schematischer Darstellung,
- Fig. 2: ein Schaubild zur Veranschaulichung des Druckgradienten bei einer Low-Flux-Hämodialyse,
- Fig. 3: ein Schaubild zur Veranschaulichung des Druckgradienten bei einer High-Flux-Hämodialyse und
- Fig. 4: ein Flußdiagramm zur Erläuterung des Verfahrens, nach dem die erfindungsgemäßen Vorrichtung arbeitet.

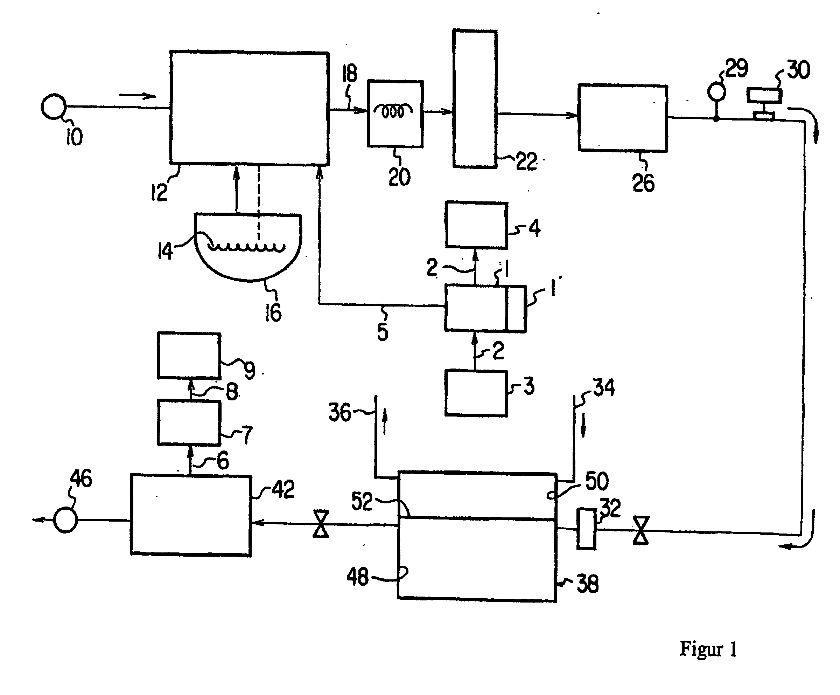

Die Hämodialysebehandlung wird nachfolgend unter Bezugnahme auf Fig. 1 beschrieben, die ein Einzelplatz-Dialysegerät in schematischer Darstellung zeigt. Das erfindungsgemäße Verfahren kann aber auch bei anderen Dialysegeräten angewandt werden, die beispielsweise einen geschlossenen Dialysierflüssigkeitskreislauf aufweisen.

Vor Beginn der Dialysebehandlung wird das gesamte System gespült und von Luftblasen befreit. Der Patient wird an das Dialysegerät angeschlossen, indem die Blutzuführleitung 34 und die Blutrückführleitung 36 mit Nadeln verbunden werden, die in eine Arterie bzw. Vene des Patienten gestochen werden. Über die Blutleitungen wird dann Blut von dem Patienten durch die Blutkammer des Dialysators 38 und zurück zu dem Patienten gepumpt.

Die Proportionierungspumpe 12 kontrolliert die Mischung der Dialysierflüssigkeit. Bei diesem Ausführungsbeispiel wird zur Herstellung der Dialysierflüssigkeit Wasser aus einem Einlaß 10 mit einem flüssigen Dialysierflüssigkeitskonzentrat 14 vermischt, das in einem Konzentratbehälter 16 bereitgestellt wird. Die Dialysierflüssigkeit 18 passiert dann eine Heizeinrichtung 20, in der die Flüssigkeit auf die Körpertemperatur erwärmt wird, bevor diese durch den Dialysator gepumpt wird. Da das einströmende Wasser Luftblasen mit sich führen kann, ist es erforderlich, der Heizeinrichtung 20 einen Luftabscheider 22 nachzuschalten. Zur Luftabscheidung der Dialysierflüssigkeit wird diese einem extrem negativen Druck, im allgemeinen 300 bis 600 mm/Hg, ausgesetzt und die Luft wird dann aus der Lösung abgeschieden.

Zur Einstellung des gewünschten Mischungsverhältnisses von Wasser und Dialysierflüssigkeitskonzentrat findet im allgemeinen ein Leitfähigkeitsmeßgerät 26 Verwendung. Die Temperatur und Leitfähigkeit der Dialysierflüssigkeit wird mittels Anzeige- und Alarmeinrichtungen 28, 30 überwacht, um sicherzustellen, daß das Dialysegerät korrekt arbeitet, bevor die Dialysierflüssigkeit dem Dialysator 38 zugeführt wird.

Ein Durchflußmeßgerät 32 überwacht die Durchflußrate der in die Dialysierflüssigkeitskammer des Dialysators 38 eintretenden Dialysierflüssigkeit. Die Bedeutung des Verhältnisses zwischen der Durchflußrate der Dialysierflüssigkeit und der Durchflußrate des Bluts in dem Dialysator für die Erfindung wird nachfolgend noch erläutert.

Die aus dem Dialysator 38 austretende Dialysierflüssigkeit passiert dann einen stromab des Dialysators angeordneten Blutleckdetektor 42, der Blut in der Dialysierflüssigkeit detektiert. Der Blutleckdetektor 42 weist im allgemeinen einen fotooptischen Sensor auf, der eine Veränderung in der optischen Durchlässigkeit mißt, die auf die Streuung des Lichts durch Hämoglobin zurückzuführen ist, das die roten Blutkörperchen in dem Blutstrom enthalten. Es kann aber auch jede andere Art der bekannten Blutleckdetektoren Verwendung finden. Die Dialysierflüssigkeit fließt schließlich aus dem Blutleckdetektor 42 in einen Abfluß 46.

Während der Hämodialysebehandlung fließt Blut über die in Fig. 1 dargestellten Blutleitungen 34, 36 von dem Patienten zu der Blutkammer des Dialysators 38 und wieder zurück zum Patienten, während frische Dialysierflüssigkeit kontinuierlich durch die Dialysierflüssigkeitskammer strömt. Der Dialysator 38 besteht aus einer Blutkammer 50, einer Dialysierflüssigkeitskammer 48 und einer semipermeablen Membran 52, die beide Kammern voneinander trennt. Die harnpflichtigen Stoffe im Blut, bei denen es sich normalerweise um kleine gelöste Substanzen handelt, können von dem die Blutkammer 50 durchströmenden Blut durch die semipermeable Membran 52 in die Dialysierflüssigkeitskammer 48 gelangen.

Die beide Kammern voneinander trennende Membran 52 ist eine semipermeable Membran mit einer Oberfläche von ca. 1 bis 2 m². Obwohl derartige Membranen in zahlreichen Ausführungsformen angeboten werden, haben derartige Membranen gemeinsam, daß diese eine Komponente einer Lösung hindurchtreten lassen und die andere Komponente daran hindern. Während der Behandlung strömt das Blut auf der Blutseite entlang der Membran, während die Dialysierflüssigkeit auf der anderen Seite fließt. Bei einer normalen Hämodialysebehandlung beträgt die Durchflußrate auf der Blutseite normalerweise 100 ml/min, während die Durchflußrate auf der Dialysierflüssigkeitsseite normalerweise in einer Größenordnung von 200 ml/min bis 500 ml/min für einen Erwachsenen liegt.

Um dem Blut Flüssigkeit zu entziehen, ist es erforderlich, auf der Blutseite 50 und der Dialysierflüssigkeitsseite 48 der Membran eine Druckdifferenz zu erzeugen. Dieser Flüssigkeitsentzug wird auch als Ultrafiltration bezeichnet.

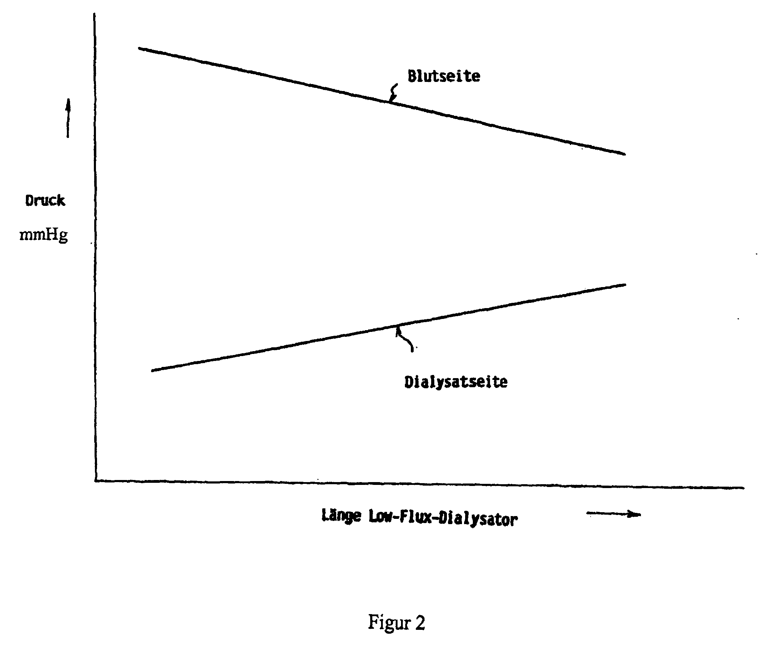

Bei der normalen Hämodialysebehandlung ist der Druckgradient in dem Dialysator derart, daß der Druck auf der Blutseite innerhalb des gesamten Dialysators positiv gegenüber dem Druck auf der Dialysierflüssigkeitsseite ist, so daß im Falle einer Leckage Blut aus der Blutkammer in die Dialysierflüssigkeitskammer gelangt. In Fig. 2 sind die Druckgradienten während einer normalen oder einer Low-Flux-Dialysebehandlung dargestellt.

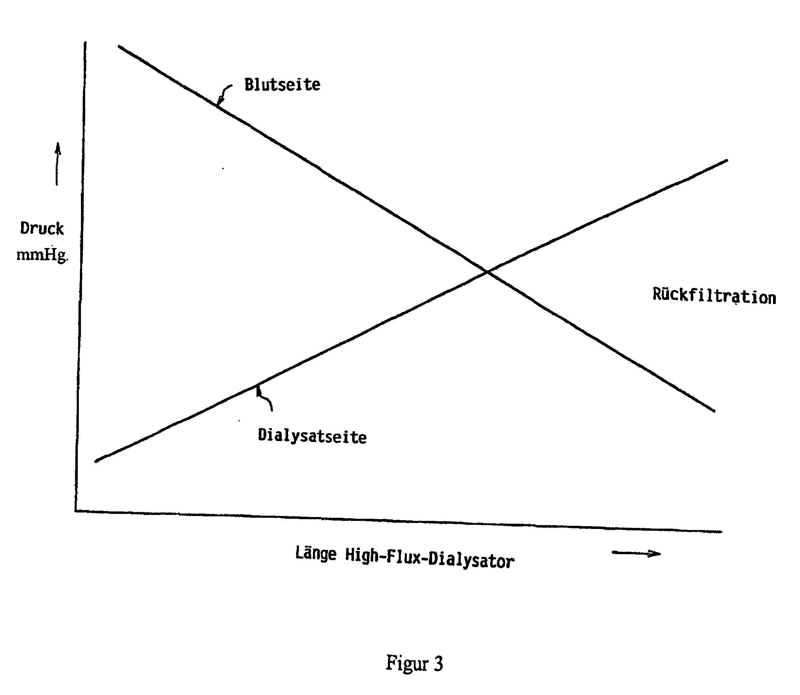

Bei verschiedenen Patienten kann es erforderlich sein, die Ultrafiltrationsrate während der Dialysebehandlung zu verändern. Es kann erforderlich sein, die Ultrafiltrationsrate zu erhöhen und die Behandlungsdauer zu verringen. In diesem Fall ist es möglich, daß der Transmembrandruck auf der Blutseite gegenüber der Dialysatseite der Membran negativ wird. In Fig. 3 ist der Druckgradient bei der High-Flux-Dialysebehandlung dargestellt.

Der in Fig. 3 dargestellte Zustand wird auch als Rückfiltration bezeichnet, weil die Dialysierflüssigkeit mittels der Dialysatormembran steril gefiltert wird und in den Blutstrom gelangt. Wenn in diesem Zustand ein Leck auftritt, gelangt Dialysierflüssigkeit von der Dialysierflüssigkeitsseite in den Blutstrom. Da in der aus dem Dialysator durch den Blutleckdetektor 42 fließenden Dialysierflüssigkeit kein Blut enthalten ist, kann das Leck nicht erkannt werden.

Wenn das Leck nicht erkannt wird, können 50 bis 100 cm³ von verunreinigtem Wasser unbemerkt in den Patienten gelangen, was zu pyrogenen Reaktionen oder eine Bakteriämie führen kann.

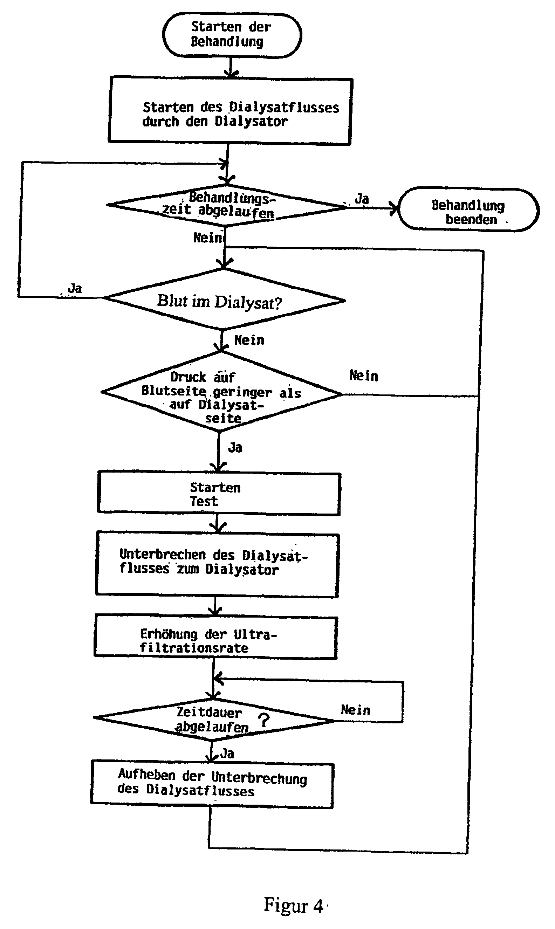

Ein bevorzugtes Ausführungsbeispiel des Verfahrens zur automatischen Überwachung der Membran von High-Flux-Dialysatoren auf Leckstellen im Bereich der Rückfiltration wird nachfolgend unter Bezugnahme auf das Flußdiagramm von Fig. 4 beschrieben. Zuerst wird mit der High-Flux-Hämodialysebehandlung des Patienten begonnen. Der Dialysierflüssigkeitsfluß wird auf einen bestimmten Wert eingestellt, wobei die Behandlung nach Ablauf einer bestimmten Behandlungszeit beendet wird. Während der Dialysebehandlung wird die Membranintegrität überwacht. Wenn der Transmembrandruck auf der Blutseite gegenüber der Dialysatseite der Membran negativ wird, wird der Dialysierflüssigkeitsfluß durch den Dialysator automatisch unterbrochen. Ansonsten wird die Behandlung fortgesetzt. Nach der Unterbrechung des Dialysierflüssigkeitsflusses wird die Ultrafiltrationsrate auf einen Wert ≥ 500 ml/min für eine Zeitdauer von mehr als 20 Sekunden erhöht. Wenn sich auf der Blutseite wieder ein positiver Druck einstellt, wird dem Dialysator wieder Dialysierflüssigkeit zugeführt. Die Dialysierflüssigkeit fließt dann aus der Maschine durch den Blutleckdetektor.

Im Falle eines Lecks in der Membran ist bei einer Ultrafiltration eine Zeitdauer von 20 Sekunden ausreichend, daß Blut durch die Membran in die Dialysierflüssigkeit gelangt. Das mit Blut angereicherte Ultrafiltrat passiert dann den Blutleckdetektor, der ein Leck anzeigt. Wenn ein Blutleck detektiert wurde, kann die im Blutkreislauf vorgesehene Blutpumpe gestoppt und der Dialysator durch eine Bypassleitung überbrückt werden.

Die Vorrichtung zur automatischen Überprüfung der Integrität der Membran des Dialysators 38 weist eine Steuereinheit 1 mit einem Zeitglied 1' auf, die über Datenleitungen 2 einerseits mit einer Einrichtung zur Überwachung des Transmembrandrucks 3 und andererseits mit der Einrichtung 4 des Hämodialysegerätes zur Einstellung der Ultrafiltrationsrate in Verbindung steht (Fig. 1). Über eine weitere Datenleitung 5 ist die Steuereinheit 1 zur Steuerung des Dialysierflüssigkeitsflusses mit der Proportionierungspumpe 12 verbunden. Über eine Datenleitung 6 ist der Blutleckdetektor 42 mit einer Auswerteinheit 7 verbunden, die über eine Datenleitung 8 mit einer Alarmeinrichtung 9 in Verbindung steht.

Die Steuereinheit 1 ist derart ausgebildet, daß der Fluß der Dialysierflüssigkeit durch den Dialysator 38 für ein durch das Zeitglied 1' vorgegebenes Zeitintervall von 20 Sekunden dann automatisch unterbrochen wird, wenn auf der Blutseite mittels der Einrichtung 3 zur Überwachung des Transmembrandrucks ein gegenüber der Dialysatseite der Membran negativer Transmembrandruck gemessen wird. Sobald die Steuereinheit 1 den Dialysierflüssigkeitsfluß unterbrochen hat, steuert diese die Einrichtung 4 zur Einstellung der Ultrafiltrationsrate derart an, daß die Ultrafiltrationsrate für den vorgegebenen Zeitraum von 20 Sekunden auf einen Wert von 500 ml/min erhöht wird. Nach Ablauf der vorgegebenen Zeitdauer setzt die Steuereinheit 1 die Ultrafiltrationsrate und den Dialysierflüssigkeitsfluß wieder auf die Normalwerte. Die Auswerteinheit 7 vergleicht das Ausgangssignal des Blutleckdetektors 42 mit einem vorgegebenen Grenzwert. Wenn das Ausgangssignal des Blutleckdetektors 42 im Falle eines Lecks über dem Grenzwert liegt, gibt diese ein Signal an die Alarmeinrichtung 9 ab, die eine Alarmfunktion auslöst. Die Alarmeinrichtung 9 kann beispielsweise die Stromversorgung zu der im Blutkreislauf angeordneten Blutpumpe unterbrechen, die in Fig. 1 nicht dargestellt ist. Bei der unter Bezugnahme auf Fig. 1 beschriebenen Ausführungsform der Vorrichtung zur Überprüfung der Membranintegrität erfolgt der Integritätstest dann, wenn eine Rückfiltration auftritt. Es ist aber auch möglich, daß der Integritätstest unabhängig von dem Transmembrandruck in vorgegebenen festen Zeitintervallen periodisch durchgeführt wird.

## Patentansprüche

1. Vorrichtung in Verbindung mit einem Hämodialysegerät mit einer Ultrafiltrationseinrichtung, um die Funktionssicherheit des Dialysators während einer Hämodialysebehandlung zu überwachen, wobei der Dialysator (38) durch eine Membran (52) in eine Blutkammer (50) und eine Dialysierflüssigkeitskammer (48) getrennt ist, und die Blutkammer in einen Blutweg und die Dialysierflüssigkeitskammer in einen Dialysierflüssigkeitsweg schaltbar sind, und wobei das Hämodialysegerät eine Einrichtung zur Einstellung der Ultrafiltrationsrate (4) und einen im Dialysierflüssigkeitsweg angeordneten Blutdetektor (42) aufweist; **dadurch gekennzeichnet, daß** eine Steuereinheit (1) mit einem Zeitglied (1') vorgesehen ist, die derart ausgebildet ist, daß der Fluß der Dialysierflüssigkeit durch die Dialysierflüssigkeitskammer (48) des Dialysators (38) für ein durch das Zeitglied vorgegebenes Zeitintervall unterbrechbar und die Ultrafiltrationsrate in dem vorgegebenen Zeitintervall auf einen vorbestimmten Wert erhöhbar ist und daß eine Auswerteinheit (7) zur Detektion eines Störfalls vorgesehen ist, die das Ausgangssignal des Blutdetektors während und/oder nach Ablauf des Zeitintervalls überwacht.

2. Vorrichtung nach Anspruch 1, **dadurch gekennzeichnet, daß** eine Einrichtung zur Messung des Drucks entlang der Membran (52) vorgesehen ist und daß die Steuereinheit (1) derart ausgebildet ist, daß der Fluß der Dialysierflüssigkeit durch die Dialysierflüssigkeitskammer (48) des Dialysators (38) dann unterbrochen wird, wenn ein negativer Transmembrandruck auf der Blutseite gegenüber der Dialysatseite der Membran auftritt.

3. Vorrichtung nach Anspruch 1, **dadurch gekennzeichnet, daß** die Steuereinheit (1) derart ausgebildet ist, daß der Fluß der Dialysierflüssigkeit in vorgegebenen Zeitintervallen periodisch unterbrochen wird.

4. Vorrichtung nach einem der Ansprüche 1 bis 3, **dadurch gekennzeichnet, daß** der Blutleckdetektor (42) stromab der Dialysierflüssigkeitskammer (48) des Dialysators (38) angeordnet ist.

## Claims

1. Device in connection with a haemodialysis unit with an ultrafiltration facility to monitor the functional reliability of the dialyser during haemodialysis treatment, whereby the dialyser (38) is separated by a membrane (52) into a blood chamber (50) and a dialysing liquid chamber (48) where the blood chamber is capable of being switched into a blood flow path and the dialysing liquid chamber is capable of being switched into a dialysing liquid flow path and whereby the haemodialysing unit has a device (4) to set the rate at which ultrafiltration occurs and a blood detector (42) arranged in the dialysing liquid flow path **characterised in that** a control unit (1) with a time function element (1') is provided which is designed in such a manner that the flow of the dialysing liquid through the dialysing liquid chamber (48) of the dialyser (38) can be interrupted for a specified time interval by means of the time function element and that the ultrafiltration rate during the specified interval is capable of being increased to a predetermined value and that an evaluation unit (7) for detecting a malfunction is provided which monitors the output signal of the blood detector during and/or after the conclusion of the time interval.

2. Device in accordance with claim 1, **characterised in that** a device for measuring the pressure across the membrane (52) is provided and that the control unit (1) is designed in such a manner that the flow of the dialysing liquid through the dialysing liquid chamber (48) of the dialyser (38) is interrupted if a negative trans-membrane pressure occurs on the blood side of the membrane compared with the dialysate side.

3. Device in accordance with claim 1, **characterised in that** the control unit (1) is designed in such a manner that the flow of dialysing liquid is interrupted periodically at predetermined intervals.

4. Device in accordance with one of the claims 1 to 3, **characterised in that** the blood detector (42) is located downstream of the dialysing liquid chamber (48) of the dialyser (38).

## Revendications

1. Dispositif en relation avec un appareil d'hémodialyse avec un dispositif d'ultrafiltration, pour surveiller la fiabilité du dialyseur pendant un traitement par hémodialyse, moyennant quoi le dialyseur (38) est séparé par une membrane (52) en une chambre sanguine (50) et une chambre de liquide de dialyse (48), la chambre sanguine peut être branchée à un canal sanguin et la chambre de liquide de dialyse peut être branchée à un canal de liquide de dialyse, moyennant quoi l'appareil d'hémodialyse comporte un dispositif pour le réglage du taux d'ultrafiltration (4) et un détecteur de sang situé dans le canal de liquide de dialyse, **caractérisé en ce qu'**une unité de commande (1) est prévue, avec un relais temporisateur (1'), qui est conçue de telle sorte que l'écoulement du liquide de dialyse à travers la chambre de liquide de dialyse (48) du dialyseur (38) peut être interrompu pendant un intervalle de temps prédéterminé par le relais temporisateur et le taux d'ultrafiltration peut être augmenté jusqu'à une valeur prédéterminée pendant l'intervalle de temps prédéterminé, et **en ce qu'**une unité d'analyse (7) est prévue pour la détection d'une perturbation, qui surveille le signal de sortie du détecteur de sang pendant et/ou après l'intervalle de temps.

2. Dispositif selon la revendication 1, **caractérisé en ce qu'**un dispositif de mesure de la pression est prévu le long de la membrane (52), et **en ce que** l'unité de commande (1) est conçue de telle sorte que l'écoulement du liquide de dialyse à travers la chambre de liquide de dialyse (48) du dialyseur (38) soit interrompu lorsqu'une pression différentielle négative règne du côté du sang par rapport au côté du dialysat de la membrane.

3. Dispositif selon la revendication 1, **caractérisé en ce que** l'unité de commande (1) est conçue de telle sorte que l'écoulement du liquide de dialyse soit interrompu périodiquement pendant des intervalles de temps prédéterminés.

4. Dispositif selon l'une des revendications 1 à 3, **caractérisé en ce que** le détecteur de sang (42) est disposé en aval de la chambre de liquide de dialyse (48) du dialyseur (38).
